(19) Europäisches Patentamt
European Patent Office
Office européen des brevets

(11) **EP 2 029 209 B1**

(12) **EUROPEAN PATENT SPECIFICATION**

(45) Date of publication and mention
of the grant of the patent:
**06.05.2020 Bulletin 2020/19**

(21) Application number: **07809334.1**

(22) Date of filing: **04.06.2007**

(51) Int Cl.:
***A61M 16/00*** *(2006.01)*

(86) International application number:
**PCT/US2007/013244**

(87) International publication number:
**WO 2007/145948 (21.12.2007 Gazette 2007/51)**

(54) **Method of using a ventilator monitor system**

METHODE ZUM BENUTZEN EINES BEATMUNGSGERÄTES

MÉTHODE POUR UTILISER UN SYSTÈME DE SURVEILLANCE DE RESPIRATEUR

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **05.06.2006 US 446660**

(43) Date of publication of application:
**04.03.2009 Bulletin 2009/10**

(73) Proprietor: **University of Florida Research
Foundation, Inc.
Gainesville, FL 32611 (US)**

(72) Inventors:
 • **BANNER, Michael, J.
  Alachua, FL 32615 (US)**
 • **EULIANO, Neil, Russell, II
  Gainesville, FL 32608 (US)**
 • **PRINCIPE, Jose, C.
  Gainesville, FL 32653 (US)**
 • **BLANCH, Paul, B.
  Gainesville, FL 32653 (US)**
 • **MELKER, Richard
  Gainsville,
  Florida (US)**

(74) Representative: **Gill Jennings & Every LLP
The Broadgate Tower
20 Primrose Street
London EC2A 2ES (GB)**

(56) References cited:
**EP-A- 1 132 106       WO-A-01/00265
WO-A-2004/019766     WO-A1-99/13932
US-A1- 2004 077 934   US-B1- 6 431 171**

## Description

Background of the Invention

Field of the Invention:

The present invention relates to the respiratory care of a patient and, more particularly, to a ventilator monitor method that receives a plurality of ventilator support signals indicative of the sufficiency of ventilation support received by the patient, receives at least one ventilator signal indicative of the level settings of the ventilator setting controls of the ventilator, and determines the desired level settings of the ventilator setting controls of the ventilator to provide the appropriate quality and quantity of ventilation support to the patient.

Background:

Mechanical ventilatory support is widely accepted as an effective form of therapy and means for treating patients with respiratory failure. Ventilation is the process of delivering oxygen to and washing carbon dioxide from the alveoli in the lungs. When receiving ventilatory support, the patient becomes part of a complex interactive system which is expected to provide adequate ventilation and promote gas exchange to aid in the stabilization and recovery of the patient. Clinical treatment of a ventilated patient often calls for monitoring a patient's breathing to detect an interruption or an irregularity in the breathing pattern, for triggering a ventilator to initiate assisted breathing, and for interrupting the assisted breathing periodically to wean the patient off of the assisted breathing regime, thereby restoring the patient's ability to breathe independently.

In those instances in which a patient requires mechanical ventilation due to respiratory failure, a wide variety of mechanical ventilators are available. Most modern ventilators allow the clinician to select and use several modes of inhalation either individually or in combination via the ventilator setting controls that are common to the ventilators. These modes can be defined in three broad categories: spontaneous, assisted or controlled. During spontaneous ventilation without other modes of ventilation, the patient breathes at his own pace, but other interventions may affect other parameters of ventilation including the tidal volume and the baseline pressure, above ambient, within the system. In assisted ventilation, the patient initiates the inhalation by lowering the baseline pressure by varying degrees, and then the ventilator "assists" the patient by completing the breath by the application of positive pressure. During controlled ventilation, the patient is unable to breathe spontaneously or initiate a breath, and is therefore dependent on the ventilator for every breath. During spontaneous or assisted ventilation, the patient is required to "work" (to varying degrees) by using the respiratory muscles in order to breathe.

The work of breathing (the work to initiate and sustain a breath) performed by a patient to inhale while intubated and attached to the ventilator may be divided into two major components: physiologic work of breathing (the work of breathing of the patient) and breathing apparatus imposed resistive work of breathing. The work of breathing can be measured and quantified in Joules/L of ventilation. In the past, techniques have been devised to supply ventilatory therapy to patients for the purpose of improving patient's efforts to breathe by decreasing the work of breathing to sustain the breath. Still other techniques have been developed that aid in the reduction of the patient's inspiratory work required to trigger a ventilator system "ON" to assist the patient's breathing. It is desirable to reduce the effort expended by the patient in each of these phases, since a high work of breathing load can cause further damage to a weakened patient or be beyond the capacity or capability of small or disabled patients. It is further desirable to deliver the most appropriate mode, and, intra-mode, the most appropriate quality and quantity of ventilation support required the patient's current physiological needs.

The early generation of mechanical ventilators, prior to the mid-1960s, were designed to support alveolar ventilation and to provide supplemental oxygen for those patients who were unable to breathe due to neuromuscular impairment. Since that time, mechanical ventilators have become more sophisticated and complicated in response to increasing understanding of lung pathophysiology. Larger tidal volumes, an occasional "sigh breath," and a low level of positive end-expiratory pressure (PEEP) were introduced to overcome the gradual decrease in functional residual capacity (FRC) that occurs during positive-pressure ventilation (PPV) with lower tidal volumes and no PEEP. Because a decreased functional residual capacity is the primary pulmonary defect during acute lung injury, continuous positive pressure (CPAP) and PEEP became the primary modes of ventilatory support during acute lung injury.

In an effort to improve a patient's tolerance of mechanical ventilation, assisted or patient-triggered ventilation modes were developed. Partial PPV support, in which mechanical support supplements spontaneous ventilation, became possible for adults outside the operating room when intermittent mandatory ventilation (IMV) became available in the 1970s. Varieties of "alternative" ventilation modes addressing the needs of severely impaired patients continue to be developed.

The second generation of ventilators was characterized by better electronics but, unfortunately, due to attempts to replace the continuous high gas flow IMV system with imperfect demand flow valves, failed to deliver high flow rates of gas in response to the patient's inspiratory effort. This apparent advance forced patient to perform excessive imposed work and thus, total work in order to overcome ventilator, circuit, and demand flow valve resistance and inertia. In recent years, microprocessors have been introduced into modern ventilators. Microprocessor ventilators are typically equipped with sensors that monitor breath-by-breath flow, pressure, volume, and derive mechanical respiratory parameters. Their ability to sense and transduce "accurately," combined with computer technology, makes the interaction between clinician, patient, and ventilator more sophisticated than ever. The prior art microprocessor controlled ventilators suffered from compromised accuracy due to the placement of the sensors required to transduce the data signals. Consequently, complicated algorithms were developed so that the ventilators could "approximate" what was actually occurring within the patient's lungs on a breath by breath basis. In effect, the computer controlled prior art ventilators were limited to the precise, and unyielding, nature of the mathematical algorithms which attempted to mimic cause and effect in the ventilator support provided to the patient.

Unfortunately, as ventilators become more complicated and offer more options, the number of potentially dangerous clinical decisions increases. The physicians, nurses, and respiratory therapists that care for the critically ill are faced with expensive, complicated machines with few clear guidelines for their effective use. The setting, monitoring, and interpretation of some ventilatory parameters have become more speculative and empirical, leading to potentially hazardous misuse of these new ventilator modalities. For example, the physician taking care of the patient may decide to increase the pressure support ventilation (PSV) level based on the displayed spontaneous breathing frequency. This may result in an increase in the work of breathing of the patient which may not be appropriate. This "parameter-monitor" approach, unfortunately, threatens the patient with the provision of inappropriate levels of pressure support.

Ideally, ventilatory support should be tailored to each patient's existing pathophysiology, rather than employing a single technique for all patients with ventilatory failure (*i.e.,* in the example above, of the fallacy of using spontaneous breathing frequency to accurately infer a patient's work of breathing). Thus, current ventilatory support ranges from controlled mechanical ventilation to total spontaneous ventilation with CPAP for support of oxygenation and the elastic work of breathing and restoration of lung volume. Partial ventilation support bridges the gap for patients who are able to provide some ventilation effort but who cannot entirely support their own alveolar ventilation. The decision-making process regarding the quality and quantity of ventilatory support is further complicated by the increasing knowledge of the effect of mechanical ventilation on other organ systems.

The overall performance of the assisted ventilatory system is determined by both physiological and mechanical factors. The physiological determinants, which include the nature of the pulmonary disease, the ventilatory efforts of the patient, and many other physiological variables, changes with time and are difficult to diagnosis. Moreover, the physician historically had relatively little control over these determinants. Mechanical input to the system, on the other hand, is to a large extent controlled and can be reasonably well characterized by examining the parameters of ventilator flow, volume, and/or pressure. Optimal ventilatory assistance requires both appropriately minimizing physiologic workloads to a tolerable level and decreasing imposed resistive workloads to zero. Doing both should insure that the patient is neither overstressed nor oversupported. Insufficient ventilatory support places unnecessary demands upon the patient's already compromised respiratory system, thereby inducing or increasing respiratory muscle fatigue. Excessive ventilatory support places the patient at risk for pulmonary-barotrauma, respiratory muscle deconditioning, and other complications of mechanical ventilation.

Unfortunately, none of the techniques devised to supply ventilatory support for the purpose of improving patient efforts to breathe, by automatically decreasing imposed work of breathing to zero and appropriately decreasing physiologic work once a ventilator system has been triggered by a patient's inspiratory effort, provides the clinician with advice in the increasingly complicated decision-making process regarding the quality and quantity of ventilatory support. As noted above, it is desirable to reduce the effort expended by the patient to avoid unnecessary medical complications of the required respiratory support and to deliver the most appropriate mode, and, intra-mode, the most appropriate quality and quantity of ventilation support required the patient's current physiological needs. Even using the advanced microprocessor controlled modern ventilators, the prior art apparatus and methods tend to depend upon mathematical models for determination of necessary actions. For example, a ventilator may sense that the hemoglobin oxygen saturation level of the patient is inappropriately low and, from the sensed data and based upon a determined mathematical relationship, the ventilator may determine that the oxygen content of the breathing gas supplied to the patient should be increased. This is similar to, and unfortunately as inaccurate as, a physician simply looking at a patient turning "blue" and determining more oxygen is needed.

From the above, in the complicated decision-making environment engendered by the modern ventilator, it is clear that it would be desirable to have a medical ventilator monitor system that alerts the clinician of the ventilator's failure to supply the appropriate quality and quantity of ventilatory support and provides advice to the clinician regarding the appropriate quality and quantity of ventilatory support that is tailored to the patient's pathophysiology. Such a ventilatory monitor system is unavailable in current systems.

WO-A-01/00265 describes a medical ventilator having a plurality of selectable ventilator setting controls governing the supply of ventilation support from the ventilator, each setting control selectable to a level setting. The ventilator preferably receives at least one ventilator setting parameter signal, each ventilator setting parameter signal indicative of the level of settings of one ventilator setting control, monitors a plurality of output signals to determine the sufficiency of the ventilation support received by the patient, and controls the level of settings of the ventilator setting controls in response to the received ventilator setting parameter signal and the output signals. The ventilator preferably utilizes a trainable neural network to determine the desired level settings of the ventilator setting controls.

WO-A-2004/019766 describes a method of creating a non-invasive predictor of both physiologic and imposed patient effort from airway pressure and flow sensors attached to the patient using an adaptive mathematical model. The patient effort is commonly measured via work of breathing, power of breathing, a pressure-time product of esophageal pressure and is important for properly adjusting ventilator support for spontaneously breathing patients.

US-B-6431171 describes an apparatus for controlling gas delivery to a patient, to maintain effective respiratory function. The apparatus includes sensors for monitoring one or more physiological variables such as breathing airflow sound, EEG, EOG, ENG, and/or patient position. The apparatus includes a determining component for deriving, from the sensed variables, data representing a respiratory event and a component for determining from the data a gas pressure or modulated gas pressure to counteract tissue vibration associated with the respiratory event, thereby tending to cancel the respiratory event. The determining component may include an algorithm adapted to generate a gas pressure signal substantially 180 degrees out of phase relative to the tissue vibration.

Summary

In accordance with a first aspect of the invention, a method according to claim 1 is disclosed.

Thus, one embodiment relates to a method of monitoring the respiratory support provided by a ventilator that is supplying a breathing gas (such as air, oxygen mixed with air, pure oxygen, *etc.*) to a patient via a breathing circuit that is in fluid communication with the lungs of the patient. The ventilator has a plurality of selectable ventilator setting controls governing the supply of ventilation support from the ventilator, each setting control selectable to a level setting. The subject system for monitoring respiratory support preferably receives at least one ventilator setting parameter signal, each ventilator setting parameter signal indicative of the level settings of one ventilator setting control, monitors a plurality of sensors, each sensor producing an output signal indicative of a measured ventilation support parameter, to determine the sufficiency of the ventilation support received by the patient, and determines the desired level settings of the ventilator setting controls in response to the received ventilator setting parameter signal and the output signals. The neural network used to determine the desired level settings of the ventilator setting controls is preferably trainable.

Detailed Description of the Figures

The accompanying drawings, which are incorporated in and constitute a part of this specification, illustrate several embodiments of the invention and together with the description, serve to explain the principals of the invention.

Fig. 1 is a block diagram of one configuration a ventilator monitor system for determining the desired ventilator control settings of a ventilator.
Fig. 2A is a block diagram of one configuration of the ventilator monitor system showing the ventilator providing ventilation support to a patient connected to the ventilator via a breathing circuit.
Fig. 2B is a block diagram of an embodiment of a ventilator monitor system showing the monitor system incorporated into the ventilator.
Fig. 3 is a block diagram of the ventilator monitor system showing a plurality of sensors connected to the processing subsystem.
Fig. 4 is a block diagram of a processing subsystem of the present invention.
Fig. 5 is a block diagram of a feature extraction subsystem of the present invention.
Fig. 6A is a block diagram of one embodiment of the intelligence subsystem of the processing subsystem.
Fig. 6B is a block diagram of a second embodiment of the intelligence subsystem of the processing subsystem.
Fig. 7 is a schematic block diagram of one realization of the system of the invention.
Fig. 8 is a diagram of the basic structure of an artificial neural network having a layered structure.

Detailed Description of the Invention

The present invention is more particularly described in the following examples that are intended to be illustrative only since numerous modifications and variations therein will be apparent to those skilled in the art. As used in the specification and in the claims, the singular form "a," "an," and "the" include plural referents unless the context clearly dictates otherwise.

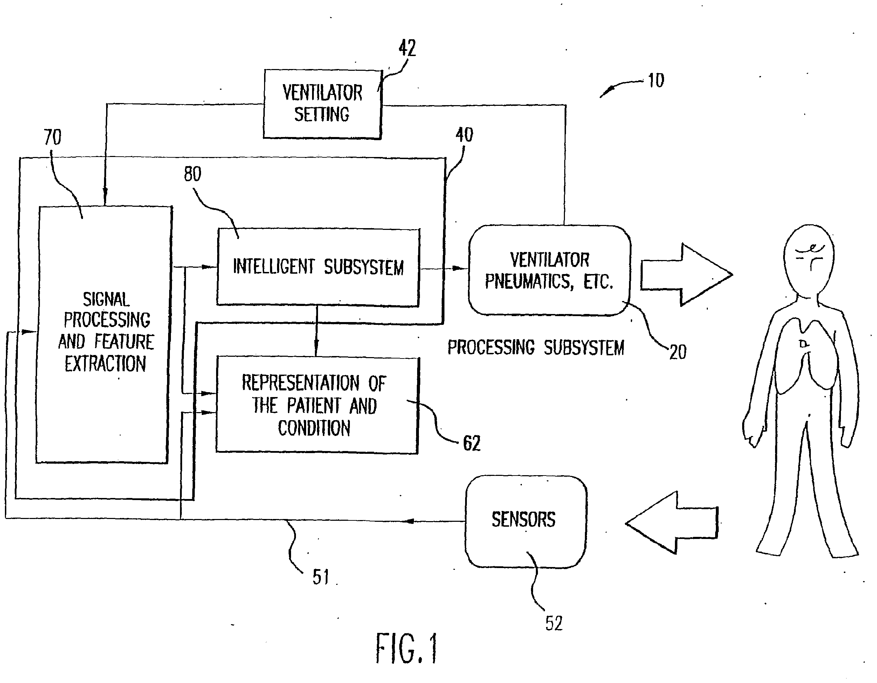

As depicted in Figs. 1 - 3, the respiratory support monitoring system **10** of the present invention preferably comprises a conventional ventilator **20,** a processing subsystem **40,** a measuring system, and a display **62.** The ventilator **20** is defined as a device that supports the patient's effort to breathe or ventilates the patient directly. These devices include, but are not limited to, critical care ventilators, transport ventilators, respiratory support devices for sleep disorders, continuous positive airway pressure (CPAP) devices, respirators for hazardous environments, and the like.

The ventilator **20** supplies a breathing gas to the lungs of the patient P via a breathing circuit **22** that typically comprises an inspiratory line **23,** an expiratory line **24,** and a patient airway access **25,** all connected by a patient connector **26**. The preferred ventilator **20** is a microprocessor-controlled ventilator of a type that is exemplified by a Mallinckrodt, Nelcor, Puritan-Bennett, 7200ae, or a Bird 6400 Ventilator. According to the present invention, the patient airway access **25** includes, but is not limited to, an endotracheal tube, laryngeal mask airway (LMA) or other supraglottic airway device such as a standard mask (oral, nasal, or full-face), nasal cannula, tracheal tube, tracheostomy or cricothyrotomy tube, and the like. Breathing gas that is supplied by the ventilator of the invention includes, but is not limited to, air, oxygen mixed with air, pure oxygen, and the like.

To control the delivery of the breathing gas, the preferred ventilator **20** typically has at least one selectable ventilator setting control **30** operatively connected to the processing system **40** for governing the supply of ventilation support provided to the patient P. As one skilled in the art will appreciate, each ventilator setting control **30** is selectable to a desired level setting. Such a ventilator **20** is particularly useful in controlling the delivery of breathing support so that the quantity and quality of ventilation support coincides with the physiological support needs of the patient P.

In the preferred embodiment, the preferred ventilator **20** can operate selectively in one or more conventional modes, as needed and selected by the operator and/or the processing subsystem **40,** including but not limited to: (i) assist control ventilation (ACMV); (ii) sychronized intermittent mandatory ventilation (SIMV); (iii) continuous positive airway pressure (CPAP); (iv) pressure-control ventilation (PCV); (v) pressure support ventilation (PSV); (vi) proportional assist ventilation (PAV); and (vii) volume assured pressure support (VAPS). Further, the level setting of one or more conventional ventilator setting controls **30** of the ventilator **20** (*i.e.,* the intra-mode setting controls of the ventilator **20)** may be adjusted, as needed and selected by the operator and/or the processing system **40** in order to maintain the sufficiency of ventilation support delivered to the patient P. The ventilator setting controls **30** of the ventilator **20** include but are not limited to controls for setting: (i) a minute ventilation (Ve) level; (ii) a ventilator breathing frequency (f) level; (iii) a tidal volume ($V_T$) level; (iv) a breathing gas flow rate (V) level; (v) a pressure limit level; (vi) a work of breathing (WOB) level; (vii) a pressure support ventilation (PSV)level; (viii) a positive end expiratory pressure (PEEP) level; (ix) a continuous positive airway pressure (CPAP) level; (x) a fractional inhaled oxygen concentration (FIO2) level; and (xi) a patient effort to breathe level.

The conventional ventilator **20** contemplated typically has a gas delivery system and may also have a gas composition control system. The gas delivery system may, for example, be a pneumatic subsystem **32** in fluid/flow communication with a gas source **34** of one or more breathing gases and the breathing circuit **22** and in operative connection with the ventilator control settings **30** of the ventilator **20** and the processing subsystem **40.** The breathing circuit **22** is in fluid communication with the lungs of the patient P. As one skilled in the art will appreciate, the pneumatic subsystem **40** of the ventilator **20** and the operative connection of that pneumatic subsystem **40** to the source of breathing gas **34** of the ventilator **20** may be any design known in the art that has at least one actuator (not shown) that is capable of being operatively coupled, preferably electrically coupled, to the ventilator setting controls **30** for control of, for example, the flow rate, frequency, and/or pressure of the breathing gas delivered by the ventilator **20** to the patient P from the gas source **34**. Such a pneumatic system **32** is disclosed in U.S. Patents Nos. 4,838,259 to Gluck et al.*,* 5,303,698 to Tobia et al.*,* 5,400,777 to Olsson et al.*,* 5,429,123 to Shaffer et al.*,* and 5,692,497 to Schnitzer et al.*,* and is exemplified by the Mallinckrodt, Nelcor, Puritan-Bennet, 7200ae, and the Bird 6400 Ventilator.

The gas composition control system may, for example, be an oxygen control subsystem **36** coupled to the source of breathing gas **34** and in operative connection to the ventilator setting controls **30** of the ventilator **20** and the processing subsystem **40**. The oxygen control subsystem **36** allows for the preferred control of the percentage composition of the gases supplied to the patient P. As one skilled in the art will appreciate, the oxygen control subsystem **36** of the ventilator **20** and the operative connection of that oxygen control subsystem **36** to the pneumatic subsystem **32** and to the source of breathing gas **34** of the ventilator **20** may be any design known in the art that has at least one actuator (not shown) that is capable of being operatively coupled, preferably electrically coupled, to the ventilator setting controls **30** for control of, for example, the percentage composition of the oxygen supplied to the patient P.

The processing subsystem **40** of the ventilator monitor system **10** preferably has an input **44** that is operatively coupled to the ventilator setting controls **30** of the ventilator **20** so that at least one ventilator setting parameter signal **42** may be received by the processing subsystem **40**. Each ventilator setting parameter signal **42** is preferably indicative of a setting of a ventilator setting control **30**. Thus, the processing system **40** is in receipt of signals **42,** preferably continuously, indicative of the current level settings of the ventilator setting controls **30**. As one skilled in the art will appreciate, the current level settings of the ventilator setting controls **30** may be stored in the memory of the processing subsystem **40.** In this example, the ventilator setting parameter signals **42** would be input from the memory of the processing subsystem **40** to the processor for continued processing and assessment.

For example, the input of the processing system **40** may receive one or more of the following ventilator setting parameter signals **42**: a minute ventilation ($V_E$) signal indicative of the $V_E$ level set on the ventilator **20**; a ventilator breathing frequency (f) signal indicative of the f level set on the ventilator **20**; a tidal volume ($V_T$) signal indicative of the $V_T$ level set on the ventilator **20**; a breathing gas flow rate (V) signal indicative of the V level set on the ventilator **20**; a pressure limit signal indicative of the pressure limit set on the ventilator **20**; a work of breathing (WOB) signal indicative of the WOB level set on the ventilator **20**; a pressure support ventilation (PSV) signal indicative of the PSV level set on the ventilator **20**; a positive end expiratory pressure (PEEP) signal indicative of the PEEP level set on the ventilator **20**; a continuous positive airway pressure (CPAP) signal indicative of the CPAP level set on the ventilator **20**; and a fractional inhaled oxygen concentration (FIO2) signal indicative of the FIO2 level set on the ventilator **20**.

The measuring system of the monitor system **10** is also operatively connected to the processing subsystem **40**. The measuring system senses and measures a plurality of ventilation support parameters which are indicative of the ventilation support provided to the patient P and the physiological condition of the patient P. It is contemplated that the measuring system may comprise at least one sensor **52**, and preferably comprises a plurality of sensors **52**, for capturing the desired ventilation support data. Each sensor **52** generates an output signal **51** based on the particular measured ventilation support parameter.

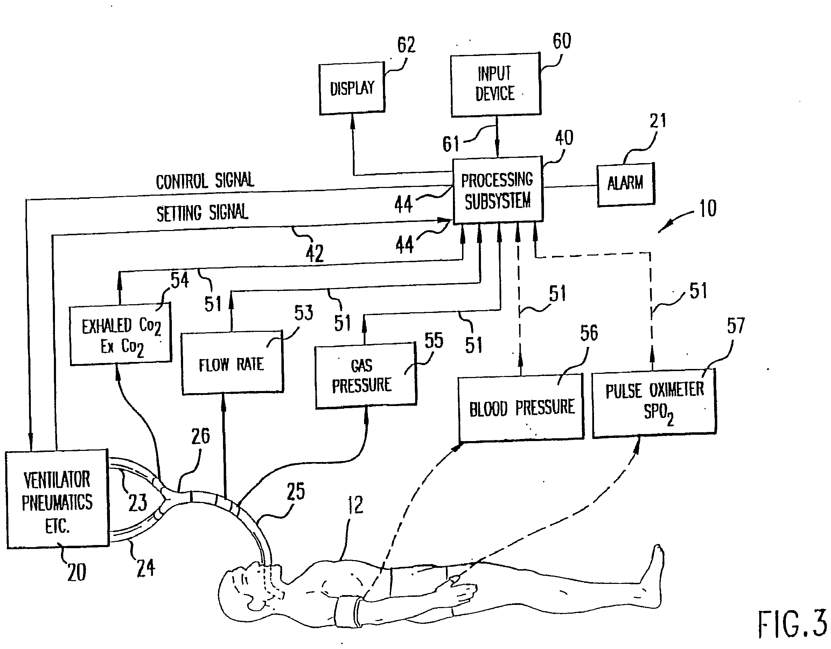

In one preferred embodiment shown in Fig. 3, the processing subsystem **30** is shown operatively connected to a flow rate sensor **53**, a exhaled CO2 (Ex CO2) sensor **54**, a pressure sensor **55**, a blood pressure sensor **56**, and a SPO2 sensor **57**. In this embodiment, it is preferred that the monitor system **10** be responsive to the output signals **51** input into the processing subsystem **40** from, for example: i) the flow rate sensor **53** which is indicative of the flow rate ventilation support parameter of the gas expired/inspired by the patient P within the breathing circuit **22**, ii) the gas pressure sensor **55** which is indicative of the pressure ventilation support parameter of the breathing gas within the breathing circuit **22**, and iii) the Ex CO2 sensor **54** which is indicative of the exhaled carbon dioxide ventilation support parameter present in the exhaled gas expired by the patient P within the breathing circuit **22** (i.e., the flow rate output signal **51** generated by the flow rate sensor **53**, the gas pressure output signal **51** generated by the gas pressure sensor **55**, and the Ex CO2 output signal **51** generated by the Ex CO2 sensor **54**). Optionally, the monitor system **10** may be responsive to output signals **51** input into the processing subsystem **40** from the output of the blood pressure sensor **56**, which in indicative of the blood pressure ventilation support parameter of the patient P, for example the arterial systolic, diastolic, and mean blood pressure of the patient P, and the SPO2 sensor **57** which is indicative of the hemoglobin oxygen saturation level ventilation support parameter of the patient P (i.e., the blood pressure output signal **51** generated by the blood pressure sensor **56** and the SPO2 output signal **51** generated by the SPO2 sensor **57**). According to the invention, information regarding the patient's blood pressure can be provided directly by the blood pressure sensor **56** (such as a blood pressure cuff) or from any one or combination of sources such as, but not limited to, an arterial line, a photoplethysmographic signal (PPG) from the SPO2 sensor **57**, pulse transit time/pulse wave velocity, and pulse pressure.

The flow rate sensor **53**, the pressure sensor **55**, and the Ex CO2 sensor **54** are preferably positioned between the patient connector **26** and the patient airway.access **25** (such as when the patient airway access is an endotracheal tube). Alternatively, it is preferred that the pressure sensor 55 be located at the tracheal end of the patient airway access **25**. The flow rate, pressure, and Ex CO2 sensors **53, 55, 54** are exemplified by Novametrics, $CO_2SMO+$ monitor (which has a flow rate, pressure and Ex CO2 sensors). The blood pressure sensor **56** and the SPO2 sensor **57** are exemplified by Dynamap, Inc's blood pressure sensor and Novametrics, $CO_2SMO+$ monitor's SPO2 sensor. The blood pressure sensor **56** and the SPO2 sensor **57** may be attached to a portion of the patient's body to render the requisite measurements. For example, an SPO2 sensor such as a pulse oximeter sensor can be placed on any portion of the body, including any area around the head (such as the ear, nose (e.g., septal, alar, or lateral nasal cartilages), cheek, tongue, forehead, neck, and the like) to obtain information from the cardiac and/or respiratory systems (such as via direct sensing from the carotid artery). Likewise, the blood pressure sensor can be placed on any portion of the body, including the arm, finger, wrist, leg, toes, and the like.

The blood pressure sensor **56**, here for example shown as a blood pressure cuff, is shown attached to the arm of the patient P and the SPO2 sensor **57**, which may, for example, be a pulse oximeter, is shown attached to a finger of the patient **12** . One skilled in the art appreciates that the blood pressure data may be derived from the SPO2 sensor **57**, which eliminates the need for the blood pressure sensor **56**.

Additional standard equipment can include an operator interface **60**, which in the preferred embodiment is a membrane keypad, a keyboard, a mouse, or other suitable input device, for providing user inputs of both data and control commands needed to execute the software which implements the various functions of the invention. The operator of the respiratory support monitoring system **10** of the present invention may provide the processing subsystem **40**, via an operator input signal generated by the operator interface **60**, with any number of applicable input parameters, such as patient identification information, patient diagnostic information, type and size of patient airway access, patient age, patient height, patient weight, or other desired patient statistics.

Such input parameters, such as patient height and weight, are useful in establishing and monitoring desired ventilator control settings and/or ventilation parameters. For example, because the size of the patient's lungs is generally a function of patient height, the optimal tidal volume (breath volume) can be associated with the patient height parameter. The normal range of tidal volumes is 6-10 mls/kg of patient weight. However, the patient's weight is typically calculated as ideal body weight which is a function of patient height (since overweight patients have the same lung size as normal or underweight patients). In addition, patient diagnostic information is also useful in establishing and monitoring desired ventilator control settings and/or ventilation parameters. This would include information concerning patient cardiac health and respiratory diseases such as chronic obstructive pulmonary disease (COPD), asthma, acute respiratory distress syndrome (ARDS), *etc.*

Accordingly, in certain embodiments of the invention, the operator provides to the processing subsystem a patient height and/or patient weight input parameter to assist in the establishment and monitoring of desired level settings of either the ventilator setting controls or ventilation parameters.

It is preferred that the operator input predetermined patient reference data, such as the arterial blood gas pH, the arterial blood gas PaO2, and/or the arterial blood gas PaCO2 of the patient's blood, and/or patient's temperature into the processing subsystem 40 as operator input signals 61 via the operator interface 60. The monitor system 10 may also be responsive to the core body temperature of the patient P which may be input into the processing subsystem 40 as an output signal 51 from a temperature sensor 58 attached to the patient P or as an operator input signal 61 via the operator interface 60.

The processing subsystem 40 preferably comprises a processor 46, for example a microprocessor, a hybrid hardware/software system, controller, or computer, and a memory. The output signals 51 and the ventilation data 72 derived from the output signals 51 are stored in the memory of the processing subsystem 40 at user-defined rates, which may be continuous, for as-needed retrieval and analysis. The ventilator setting signal 42 may also be stored in the memory at a user-defined rate. As one skilled with the art will appreciate, any generated signal may be stored in the memory at user-defined rates. The memory may be, for example, a floppy disk drive, a CD drive, internal RAM or hard drive of the associated processor 12.

The processing subsystem 40 is responsive to the output signals 51 of the measuring means, the ventilator setting parameter signal(s) 42, and, if provided, the operator input signals 61. The processor 46 runs under the control of a program stored in the memory and has intelligent programming for the determination of at least one desired level setting of the ventilator setting controls 30 based on at least a portion of the output signal 51 from the measuring means, at least a portion of the ventilator setting parameter signal(s) 42 received at the input 44 of the processing subsystem 40, and, if provided, at least a portion of the operator input signals 61.

The desired level settings for the ventilator setting controls 30 of the ventilator 20 may include at least one of the group of: i) a minute ventilation ($V_E$) level indicative of the desired $V_E$ level to set on the ventilator 20; ii) a ventilator breathing frequency (f) level indicative of the desired f level to set on the ventilator 20; iii) a tidal volume ($V_T$) level indicative of the $V_T$ level to set on the ventilator 20; iv) a breathing gas flow rate (V) level indicative of the V level to set on the ventilator 20; v) a pressure limit level indicative of the pressure limit level to set on the ventilator 20; vi) a work of breathing (WOB) level indicative of the WOB level to set on the ventilator 20; vii) a pressure support ventilation (PSV) level indicative of the PSV level to set on the ventilator 20; viii) a positive end expiratory pressure (PEEP) level indicative of the PEEP level to set on the ventilator 20; ix) a continuous positive airway pressure (CPAP) level indicative of the CPAP level to set on the ventilator 20; and x) a fractional inhaled oxygen concentration (FIO2) level indicative of the FIO2 level to set on the ventilator 20.

The desired level setting of the ventilator setting controls 30 determined by the processing system 40 of the monitor system 10 may be displayed to the operator via the display. The display of the monitor system 10 preferably comprises a visual display 62 or CRT, electronically coupled to the processing subsystem 40 for outputting and displaying output display signals generated from the processing subsystem 40.

Still further, the monitor system 10 may have an alarm 21 for alerting the operator of either a failure of the monitor system 10, such as a power failure of loss of signal data input, or an inappropriate setting of a ventilator control 30, such as a level setting of a ventilator setting control 30 currently controlling the delivery of ventilator support to the patient P differing from a recommended desired level setting of the ventilator setting control 30. Preferably, the alarm 21 comprises a visual and/or audio alarm, but any means for alerting the operating clinician know to one skilled in the art may be used. Of course, it is desired to use a backup power supply, such as a battery.

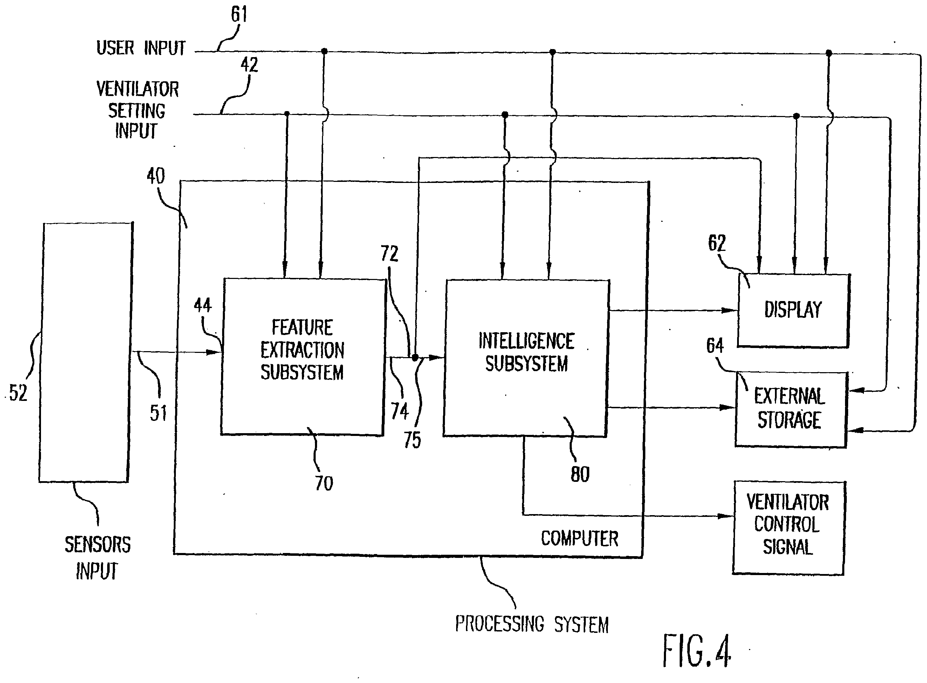

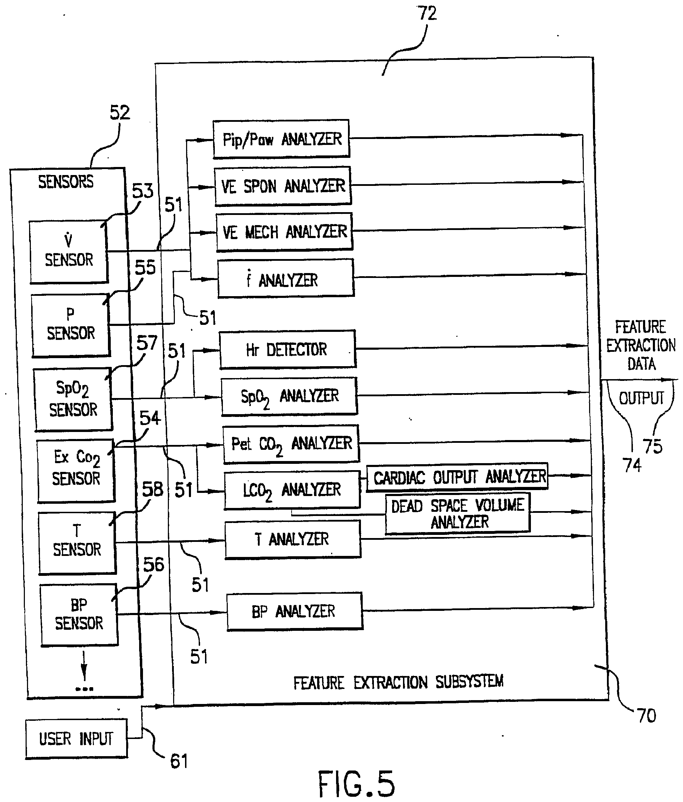

Referring to Figs. 4 and 5, the processing subsystem of the preferred embodiment of the present invention has a means for determining the desired ventilation control settings 30 of the ventilator 20. The determining means preferably comprises a feature extraction subsystem 70 and an intelligence subsystem 80. The feature extraction subsystem 70 has a means for extracting and compiling pertinent ventilation data features from the input of the measuring means (*i.e.,* the output signals 51). In effect, the feature extraction subsystem 70 acts as a preprocessor for the intelligence subsystem 80. An example of the feature extraction subsystem 70 is shown in Fig. 5. Here, a flow rate sensor 53, a gas pressure sensor 55, a SPO2 sensor 57, an Ex CO2 sensor 54, a temperature (T) sensor **58,** a blood pressure (BP) sensor **56,** of a type described above, and any other desired sensor are operatively connected to the feature extraction subsystem **70** of the processing subsystem **40.** Preferably, the flow rate sensor **53,** the gas pressure sensor **55,** and the Ex CO2 sensor **54** provide the only inputs to the monitor system. The other sensor inputs, and the user input, may be included to increase the reliability and confidence of the determined desired level settings of the controls **30.** The monitor system **10** preferably adjusts the extraction of ventilator data **72** as a function of the presence or absence of these optional inputs. By making the number of inputs optional, which also makes the required number of sensors **52** comprising the measuring system optional, the number of environments in which the ventilator monitor system 10 can be used is increased.

The purpose of the feature extraction subsystem **70** is to calculate and/or identify and extract important variables or features from the output signals **51** produced by the measuring means. For example, from the exemplified required inputs to the feature extraction subsystem **70,** *i.e.,* the gas pressure output signal **51,** the flow rate output signal **51,** and the Ex CO2 output signal **51,** a plurality of ventilation data **72** may be derived. The derived ventilation data **72** may comprise: the values of any output signals **51** used, such as, for example, the gas pressure output signal **51,** the flow rate output signal **51,** and the Ex CO2 output signal **51** output signals **51;** the peak inflation pressure (PIP), which is the maximal pressure generated during mechanical ventilation of the lungs; the mean airway pressure (PAW), which is the average positive pressure measured at the airway opening in the patient airway access **25** (such as when the patient airway access is an endotracheal tube) or in the breathing circuit **22** over one minute; the positive end expiratory pressure (PEEP), which is the baseline or starting positive pressure prior to mechanical inflation or the positive pressure applied continuously during inhalation and exhalation during spontaneous ventilation; breathing frequency (f), which is the frequency or rate or breathing per minute (the total breathing frequency $f_{TOT}$ is the sum of the mechanical $f_{MECH}$ ventilator preselected frequency and the spontaneous $f_{SPON}$ patient breathing frequency); the tidal volume ($V_T$), which is the volume of the breathing gas moving in and out of the lungs per breath ($V_{T\ MECH}$ is the ventilator preselected $V_T$ per breath and $V_{T\ SPON}$ is the.inhaled and exhaled volume per breath of the patient); the minute exhaled ventilation (VE), which is the volume of breathing gas moving in and out of the lungs of the patient per minute ($V_E$ is the product of the breathing frequency f and the tidal volume ($V_E = f \times V_T$), and the $V_{E\ TOT}$ is the sum of the ventilator preselected $V_E$ ($V_{E\ MECH}$) and the spontaneous patient $V_E$ inhaled and exhaled per minute ($V_{E\ SPON}$)); the inhalation-to-exhalation time ratio (I:E ratio), which is the ratio of inhalation time to exhalation time during mechanical ventilation; the physiologic dead space volume ($V_{Dphys}$),

which is the volume of gas in the anatomic airway and in ventilated, unperfused alveoli that does not participate in blood gas exchange; the lung carbon dioxide elimination rate (LCO2), which is the volume of CO2 exhaled per breath or per minute (LCO2 is the area under the Ex CO2 and volume curve); the partial pressure end-tidal carbon dioxide level (PetCO2), which is the partial pressure of the exhaled CO2 measured at the end of the exhalation; the cardiac output (CO) of the patient, which is the amount of blood ejected from the heart per minute and which may, for example be derived from the determined LCO2 rate; the respiratory system compliance and resistance; the pressure-volume loops; and the respiratory muscle pressure or the patient effort to breathe.

The patient effort to breathe can be quantified in many ways, including but not limited to: work of breathing, which quantifies the normalized effort required by the patient to take a single breath, typically expressed in Joules per liter; power of breathing, which quantifies the effort required by the patient to breath for 1 minute, typically expressed in Joules; and the pressure time product, which quantifies the patient effort per minute by summing the area under/above the pleural/esophageal pressure curve and typically expressed in cm H20 per minute. These estimates of patient effort may be derived from, but not limited to, the determined respiratory muscle pressure, esophageal pressure tracings, airway pressure, flow, and volume traces, CO2 traces, SPO2 traces, and parameters derived thereof. Such quantified or estimated values for the patient effort to breathe can be provided as a patient effort to breathe signal for use in accordance with the present invention.

It is often desirable to control a patient's required effort to breathe to maintain the patient's comfort and respiratory strength. If the ventilator is providing too much support, the patient will not be required to use adequate muscle activity to breathe and the muscles may atrophy. Likewise, if the ventilator is not providing enough support, the patient may become fatigued and not be able to support his own breathing any longer. In addition, there may be times when it is desirable to rest or exercise the patient for certain medical conditions or weaning. Knowing the patient effort allows the system of the invention to better recommend changes in the ventilator parameters. Further, the quantified patient effort to breathe (such as communicated via the patient effort to breathe signal) can be used to establish and/or monitor desired level settings for ventilator controls. For example, in one embodiment of the invention, the patient effort to breathe signal is evaluated by a processing subsystem of the invention for use in determining the desired setting of at least one parameter and/or ventilator setting control.

Ventilation data **72** may also be derived from the exemplified optional inputs to the feature extraction subsystem **70**. From the SPO2 output signal **51** (such as a pulse oximeter), the arterial blood hemoglobin oxygen saturation level and the heart rate may be determined, and the pulsatile blood pressure waveform of the SPO2 output signal **51,** such as a plethysmographic (PPG) signal), are used to establish and monitor desired settings for ventilator control(s) and/or ventilation parameters to optimize patient oxygenation without sacrificing cardiac output.

There are many known methods for assessing cardiac output. For example, Adolph Fick's measurement of cardiac output, first proposed in 1870, has served as the standard by which all other means of determining cardiac output have been evaluated since that date. Fick's well-known equation, written for $CO_2$, is:

$$Q = \frac{V_{CO2}}{(C_{VCO2} - C_{aCO2})}$$

where Q is cardiac output, $V_{CO2}$ is the amount of $CO_2$ excreted by the lungs and $C_{aCO2}$ and $C_{VCO2}$ are the arterial and venous $CO_2$ concentrations, respectively.

Expired $CO_2$ levels can be monitored to estimate arterial $CO_2$ concentrations and a varied form of the Fick Equation can be applied to evaluate observed changes in expired $CO_2$ to estimate cardiac output. Use of the Fick Equation to determine cardiac output in non-invasive procedures requires the comparison of a "standard" ventilation event to a sudden change in ventilation which causes a change in expired $CO_2$ values and a change in excreted volume of $CO_2$. Other methods for assessing cardiac output that can be used in accordance with the subject invention include those disclosed in U.S. Patent No- 6,648,831.

The PPG signal from the SPO2 output signal is used to determine arterial blood pressure as well as assist (with other output and/or input signals) in determining whether the PEEP signal is at a desired setting for appropriate patient oxygenation. The PPG signal is used by the processing subsystem in place of input from the blood pressure sensor in optimizing the PEEP ventilation parameter and/or patient oxygenation without sacrificing cardiac output.

Typically oxygenation is optimized by adjusting the fraction of inspired O2 (FIO2) delivered by the ventilator and by adjusting PEEP. Increasing FIO2 can increase patient oxygenation, but FIO2 settings much above room air (21 %) can eventually be toxic to the patient. Increasing PEEP can also increase patient oxygenation, typically by holding open sick lungs to prevent lung and alveolar collapse, thus allowing for better gas exchange between the lungs and circulatory system. If PEEP is too high (and this value varies by patient), then the increased lung pressure can reduce the amount of blood flowing back to the lungs and heart because of the increased pressure gradient between the lungs and the rest of the body. This decreased venous return can reduce cardiac output leading to decreased blood pressure and poor patient blood flow.

The PPG signal is known to contain: (1) a pulsatile signal created by blood pulsing through the arteries and veins with each heart beat; and (2) a baseline (but varying) offset that this pulsatile signal modulates (or "rides on"). Both the baseline offset and pulsatile signals are affected by breathing and/or intrathoracic pressure.

As described above, intrathoracic pressure (pressure in the chest, often driven by pressure in the lungs) can change both venous impedance (impedance of the blood returning to the lungs/heart via the veins) and cardiac output (the volume of blood ejected by the heart each beat). This is commonly seen in arterial pressure waveforms but is also known to exist in the PPG. For instance, the PPG pulsatile waveform varies with cardiac output since less blood pumped by the heart creates smaller PPG peaks and vice-versa. Also, increased baseline intrathoracic pressure will increase venous impedance which will increase the amount of blood pooling in the veins. This will cause a change (decreased signal strength) in the baseline signal of the PPG that varies with the breathing and intrathoracic pressure. Therefore, from the PPG, signals indicative of intrathoracic pressure and its affects on the respiratory and cardiac system may be determined. Examples include, but are not limited to: patient effort (which includes parameters such as power of breathing, work of breathing, *etc.*); the effect of intrathoracic pressure on cardiac output (*e.g.* excessively high PEEP); and increasing changes in intrathoracic pressure during breathing that may be caused by deterioration of lung function.

Additionally, from the blood pressure output signal **51,** the arterial systolic, diastolic and mean blood pressure of the patient P may be determined. Further, from the temperature output signal **51,** the core body temperature of the patient may **12** be derived. Still further, from the arterial blood hemoglobin oxygen saturation level and the determined LCO2, the dead space volume may be determined.

In certain embodiments, the cardiac output can be derived from the Ex CO2 output signal **51** to the feature extraction subsystem. The cardiac output from the Ex CO2 output signal is used (in conjunction with the plethysmography output signal) to determine whether the PEEP signal is set appropriately to optimize patient oxygenation without sacrificing cardiac output.

The feature extraction subsystem **70** may also receive user input via the operator interface **60** and may receive the ventilator setting parameter signal **42.** The ventilation data **72** is preferably compiled in the feature extraction subsystem **70** and a feature vector **74** or matrix is preferably generated which contains all of the ventilation data items used by the monitor subsystem **10** to perform the ventilation support assessment process. The feature vector **74** may be updated at user-defined intervals such as, for example, after each breath or each minute and is output from the feature extraction subsystem **70** to the intelligence subsystem **80** as a ventilation data output signal **75.** Alternatively, as one skilled in the art will appreciate, the ventilation data **72** may be directly outputted to the intelligence subsystem **80** as the ventilation data output signal **75** without the intervening step of generating the feature vector **74** or matrix. The ventilation data **72** may also be outputted to the display **62**.

In certain embodiments, the processing subsystem **40** has the ability to evaluate the time history (or trend) of input and/or output signals (such as evaluating the input and output signals throughout a period of time where the period may be short-term or long-term). According to the subject invention, the trend can also be used to determine when physiologically significant events might be occurring, rather than normal patient variation. Noted changes in trend could be a reflection of triggered changes in parameters from a known baseline rather than absolute values of the parameters. Also, the slope of the trend (how quickly the parameters change) could determine how best to adjust the ventilator control(s) and/or parameter(s) to desired settings. Thus, the trend of input and/or output signals can be used to determine a desired setting for ventilation control(s) and/or ventilation parameter(s).

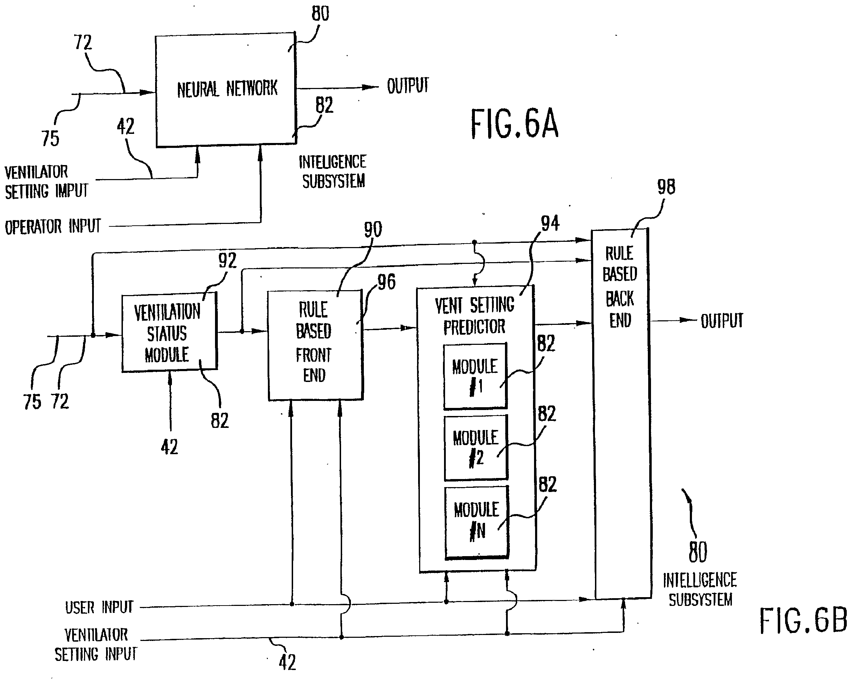

Referring to Figs. 4, 6A and 6B, the intelligence subsystem **80** of the processing subsystem **40** preferably has a neural network **82**. The primary function of the intelligence subsystem **80** is to make an assessment of the ventilator support provided to the patient and, based upon the assessment, recommend the desired level settings of the ventilator setting controls **30** which will adequately, and preferably optimally, support the physiological ventilation support needs of the patient P. For example, as shown in Fig. 6A, the intelligence subsystem **80** of the processing subsystem **40** may have a neural network **82** that receives the ventilation data output signal **75** containing the compiled ventilation data **72**. The neural network **82** also receives the ventilator setting parameter signal **42** and may receive user input from the operator interface **60**.

To fully appreciate the various aspects and benefits produced by the present invention, a basic understanding of neural network technology is required. Following is a brief discussion of this technology, as applicable to the ventilator monitor system **10** and method of the present invention.

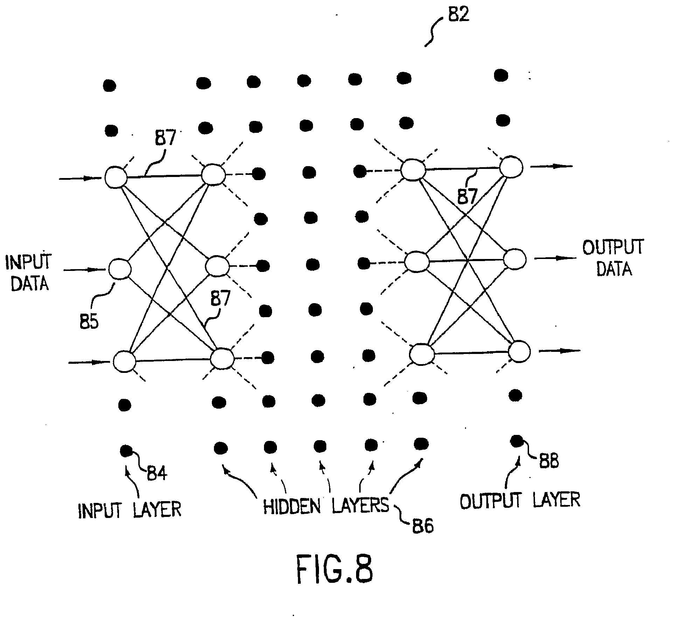

Artificial neural networks loosely model the functioning of a biological neural network, such as the human brain. Accordingly, neural networks are typically implemented as computer simulations of a system of interconnected neurons. In particular, neural networks are hierarchical collections of interconnected processing elements configured, for example, as shown in FIG. 8. Specifically, FIG. 8 is a schematic diagram of a standard neural network **82** having an input layer **84** of processing elements, a hidden layer **86** of processing elements, and an output layer **88** of processing elements. The example shown in FIG. 8 is merely an illustrative embodiment of a neural network **82** that can be used in accordance with the present invention. Other embodiments of a neural network **82** can also be used, as discussed next.

Turning next to the structure of a neural network **82,** each of its processing elements receives multiple input signals, or data values, that are processed to compute a single output. The output value is calculated using a mathematical equation, known in the art as an activation function or a transfer function that specifies the relationship between input data values. As known in the art, the activation function may include a threshold, or a bias element. As shown in FIG. 8, the outputs of elements at lower network levels are provided as inputs to elements at higher levels. The highest level element, or elements, produces a final system output, or outputs.

In the context of the present invention, the neural network **82** is a computer simulation that is used to produce a recommendation of the desired ventilator setting of the ventilator controls 30 of the ventilator **20** which will adequately, and preferably optimally, support the physiological ventilation support needs of the patient, based upon at least a portion of the available ventilation setting parameters **42** and at least a portion of the ventilation data output signal **75** (*i.e.,* at least a portion of the derived ventilation data 72).

The neural network **82** of the present invention may be constructed by specifying the number, arrangement, and connection of the processing elements which make up the network **82.** A simple embodiment of a neural network **82** consists of a fully connected network of processing elements. The processing elements of the neural network **82** are grouped into layers: an input layer **84** where at least a portion of selected ventilation data **72,** output signals **51,** and the selected ventilator setting parameter signals **42** are introduced; a hidden layer **86** of processing elements; and an output layer **88** where the resulting determined level setting(s) for the control(s) **30** is produced. The number of connections, and consequently the number of connection weights, is fixed by the number of elements in each layer.

In a preferred embodiment of the present invention, the data types provided at the input layer may remain constant. In addition, the same mathematical equation, or transfer function, is normally used by the elements at the middle and output layers. The number of elements in each layer is generally dependent on the particular application. As known in the art, the number of elements in each layer in turn determines the number of weights and the total storage needed to construct and apply the neural network **82**. Clearly, more complex neural networks **82** generally require more configuration information and therefore more storage.

In addition to the structure illustrated in FIG. 6A, the present invention contemplates other types of neural network configurations for the neural network module such as the example shown in Fig. 6B, which is described in more detail below. All that is required by the present invention is that a neural network **82** be able to be trained and retrained, if necessary, for use to determine the desired level settings of the controls 30 of the ventilator **20.** It is also preferred that the neural network **82** adapt (*i.e.,*

learn) while in operation to refine the neural network's 82 determination of the appropriate level settings for the controls 30 of the ventilator **20.**

Referring back to Figs. 6A and 8, the operation of a specific embodiment of a feedforward neural network **82** is described in more detail. It should be noted that the following description is only illustrative of the way in which a neural network **82** used in the present invention can function. Specifically, in operation, at least a portion of selected ventilation data **72** from the ventilation data output signal **75** and the selected ventilator setting parameter signals **42** (*i.e.,* collectively the input data) is provided to the input layer **84** of processing elements, referred to hereafter as inputs. The hidden layer elements are connected by links **87** to the inputs, each link **87** having an associated connection weight. The output values of the input processing elements propagate along these links **87** to the hidden layer **86** elements. Each element in the hidden layer **86** multiplies the input value along the link **87** by the associated weight and sums these products over all of its links **87.** The sum for an individual hidden layer element is then modified according to the activation function of the element to produce the output value for that element. In accordance with the different embodiments of the present invention the processing of the hidden layer elements can occur serially or in parallel.

If only one hidden layer **86** is present, the last step in the operation of the neural network is to compute the output(s), or the determined level setting(s) of the control(s) 30 of the ventilator by the output layer element(s). To this end, the output values from each of the hidden layer processing elements are propagated along their links **87** to the output layer element. Here, they are multiplied by the associated weight for the link **87** and the products are summed over all links **87.** The computed sum for an individual output element is finally modified by the transfer function equation of the output processing element. The result is the final output or outputs which, in accordance with a preferred embodiment of the present invention, is the desired level setting or settings of the ventilator setting controls 30.

In the example of the intelligence subsystem 80 shown in Fig. 6B, the intelligence subsystem **80** is a hybrid intelligence subsystem that contains both rule-based modules **90** as well as neural networks **82.** In this alternative embodiment of the intelligence subsystem **90,** the determination of the desired level settings of the controls **30** of the ventilator **20** are broken down into a number of tasks that follow classical clinical paradigms. Each task may be accomplished using a rule-based system **90** or a neural network **82.** In the preferred configuration, the determination of desired level settings of the ventilator setting controls **30** are performed by one of a series of neural networks **82.**

The purpose of the ventilation status module **92** is to make an initial assessment of the adequacy of the ventilation support being provided to the patient P based on the level settings of the ventilator setting controls 30

(as inputted to the intelligence subsystem by the ventilator setting parameter signals **42)** and the ventilation data output signal. The final determination of the desired level settings of the ventilator setting controls **30** is accomplished by one of a series of available neural networks **82** in the ventilator control setting predictor module **94.** The purpose of the rule-based front end **96** is to determine, based on inputs from the ventilation status module **92,** data entered by the operator, and the ventilator setting parameter signal **42,** which of the available neural networks **82** will determine the desired level settings of the ventilator setting controls 30. The rule-based front end **96** will also determine which inputs are extracted from the ventilation data output signal **75** and presented to the selected neural network **82.** Inputs to the ventilator control setting predictor module **94** include ventilator data **72** from the ventilation data output signal **75,** user input, and input from the ventilator setting parameter signals **42.** The purpose of the rule-based back end module **98** is to organize information from previous modules, neural networks **82,** user input, and ventilation data **72** in the ventilation data output signal and to format the information for display on the visual display **62** as well as for storage to an external storage **64** such as a disk file.

As with most empirical modeling technologies, neural network development requires a collection of data properly formatted for use. Specifically, as known in the art, input data and/or the outputs of intermediate network processing layers may have to be normalized prior to use. It is known to convert the data to be introduced into the neural network **82** into a numerical expression, to transform each of the numerical expressions into a number in a predetermined range, for example by numbers between 0 and 1. Thus, the intelligent subsystem of the present invention preferably has means for: i) selecting at least a portion of the ventilation data **72** from the ventilation data output signal **75** and at least a portion of the ventilator setting parameter signals **42,** ii) converting the selected portion of the ventilation data **72** and the selected portion of the ventilator setting parameter signals **42** into numerical expressions, and iii) transforming the numerical expressions into a number in a predetermined range.

In one conventional approach which can also be used in the present invention, the neural network **82** of the present invention may include a preprocessor **83.** The preprocessor **83** extracts the correct data from the processing subsystem memory **48** and normalizes each variable to ensure that each input to the neural network **82** has a value in a predetermined numerical range. Once the data has been extracted and normalized, the neural network **82** is invoked. Data normalization and other formatting procedures used in accordance with the present invention are known to those skilled in the art and will not be discussed in any further detail.

In accordance with a preferred embodiment of the present invention the neural network **82** is trained by being provided with the ventilator control setting assessment made by a physician and with input data, such as ventilation data **72,** the ventilation control setting parameter signals **42,** and the output signals **51** that were available to the physician. In the sequel, the assessment along with the corresponding input measurement and input data is referred to as a data record. All available data records, possibly taken for a number of different patients, comprise a data set. In accordance with the present invention, a data set corresponding is stored in memory and is made available for use by the processing subsystem 40 for training and diagnostic determinations.

A typical training mechanism used in a preferred embodiment of the present invention is briefly described next. Generally, the specifics of the training process are largely irrelevant for the operation of the ventilation monitor system. In fact, all that is required is that the neural network **82** be able to be trained and retrained, if necessary, such that it can be used to determine acceptably accurate determinations of desired level settings of the controls 30 of the ventilator **20.** Neural networks **82** are normally trained ahead of time using data extracted from patients **12** by other means. Using what it has learned from the training data, the neural network 82 may apply it to other/new patients P.

As known in the art, a myriad of techniques has been proposed in the past for training feedforward neural networks. Most currently used techniques are variations of the well-known error back-propagation method. The specifics of the method need not be considered in detail here. For further reference and more detail the reader is directed to the excellent discussion provided by Rumelhardt et al. in "Parallel Distributed Processing: Explorations in the Microstructure of Cognition," vols. 1 and 2, Cambridge: MIT Press (1986), and "Explorations in Parallel Distributed Processing, A Handbook of Models, Programs, and Exercises,".

Briefly, in its most common form back-propagation learning is performed in three steps:

    1. Forward pass;
    2. Error back-propagation; and
    3. Weight adjustment.

As to the forward pass step, in accordance with the present invention a single data record, which may be extracted from the ventilation data output signal **75** and the ventilator setting parameter signal(s) **42,** is provided to the input layer **84** of the network **82.** This input data propagates forward along the links **87** to the hidden layer elements which compute the weighted sums and transfer functions, as described above. Likewise, the outputs from the hidden layer elements are propagated along the links to the output layer elements. The output layer elements computes the weighted sums and transfer function equations to produce the desired ventilator control settings **30.**

In the following step of the training process, the physician assessment associated with the data record is made available. At that step, the determination of the desired level settings of the ventilator controls **30** produced by the neural network **82** is compared with the physician's assessment. Next, an error signal is computed as the difference between the physician's assessment and the neural network's **82** determination. This error is propagated from the output element back to the processing elements at the hidden layer **86** through a series of mathematical equations, as known in the art. Thus, any error in the neural network output is partially assigned to the processing elements that combined to produce it.

As described earlier, the outputs produced by the processing elements at the hidden layer **86** and the output layer 88 are mathematical functions of their connection weights. Errors in the outputs of these processing elements are attributable to errors in the current values of the connection weights. Using the errors assigned at the previous step, weight adjustments are made in the last step of the back-propagation learning method according to mathematical equations to reduce or eliminate the error in the neural network determination of the desired level setting of the ventilator setting controls **30.**

The steps of the forward pass, error back-propagation, and weight adjustment are performed repeatedly over the records in the data set. Through such repetition, the training of the neural network **82** is completed when the connection weights stabilize to certain values that minimize, at least locally, the determination errors over the entire data set. As one skilled in the art will appreciate however, the neural network **82** may, and preferably will, continue to train itself (*i.e.,* adapt itself) when placed into operational use by using the data sets received and stored in the memory of the processing subsystem 40 during operational use. This allows for a continual refinement of the monitor **10** as it is continually learning, *i.e.,* training, while in operational use. Further, it allows for the continual refinement of the determination of the appropriate ventilator level settings in regard to the particular patient P to which the ventilator **20** is operatively attached.

In addition to back-propagation training, weight adjustments can be made in alternate embodiments of the present invention using different training mechanisms. For example, as known in the art, the weight adjustments may be accumulated and applied after all training records have been presented to the neural network **82.** It should be emphasized, however, that the present invention does not rely on a particular training mechanism. Rather, the preferred requirement is that the resulting neural network **82** produce acceptable error rates in its determination of the desired level settings of the ventilator setting controls **30.**

Upon completion of the determination of the desired level settings of the ventilator setting controls **30** by the intelligent subsystem **80** of the processing system **40,** the desired level settings of the ventilator setting controls **30** may be displayed on the visual display **62** for use by the physician. The stored ventilation data output signal **75,** and particularly the subset of the ventilation data output signal **75** containing the ventilation data **72** that was used by the intelligent subsystem 80 in the determination of the desired level setting of the controls **30,** may be provided to the visual display **62.** Also, the stored ventilator setting parameter signals **42** and the stored output signals **51** may be displayed on the visual display **62** in an appropriate format. At this point, the physician can review the results to aid in her or his assessment of the desireablity of the recommended desired level settings of the ventilator setting controls **30.** The displayed results can be printed on printer [not shown] to create a record of the patient's condition. In addition, with a specific preferred embodiment of the present invention, the results can be communicated to other physicians or system users of computers connected to the ventilator monitor system 10 via an interface (not shown), such as for example a modem or other method of electronic communication.

Additionally, a preferred embodiment the present invention provides a real-time ventilator monitor system **10** and method. Real-time operation demands, in general, that input data be entered, processed, and displayed fast enough to provide immediate feedback to the physician in the clinical setting. In alternate embodiments, off-line data processing methods can be used as well. In a typical off-line operation, no attempt is made to respond immediately to the physician. The measurement and interview data in such case is generated some time in the past and stored for retrieval and processing by the physician at an appropriate time. It should be understood that while the preferred embodiment of the present invention uses a real-time approach, alternative embodiments can substitute off-line approaches in various steps.

The preferred method of operation of the present invention comprises the steps of receiving at least one ventilator setting parameter signal **42** indicative of the current level settings of the controls **30** of the ventilator **20,** monitoring a plurality of output signals **51** to determine the sufficiency of ventilation support supplied to the patient P, determining the desired level settings of the ventilator setting controls **30,** and displaying the desired level settings of the controls **30** to the operating clinician.

The output signals **51** received may comprise a plurality of signals selected from a group of: an exhaled carbon dioxide signal indicative of the exhaled carbon dioxide (ExCO2) level of the exhaled gas expired by the patient P within the breathing circuit 22; a flow rate signal indicative of the flow rate (V) of the inhaled/exhaled gas expired by patient P within the breathing circuit **22;** a pulse oximeter hemoglobin oxygen saturation (SpO2) signal indicative of the oxygen saturation level of the patient P; a pressure (P) signal indicative of the pressure of the breathing gas within the breathing circuit **22;** a blood pressure (BP) signal indicative of the blood pressure of the patient **12 .** The output signals **51** may also comprise a temperature (T) signal indicative of the core body temperature of the patient P, an arterial blood gas PaO2 signal, an arterial blood gas PaCO2 signal, and/or an arterial blood gas pH signal..

The ventilator setting parameter signal **42** may comprise at least one of: a minute ventilation ($V_E$) signal indicative of the $V_E$ level set on the ventilator **20;** a ventilator breathing frequency (f) signal indicative of the f level set on the ventilator **20;** a tidal volume ($V_T$) signal indicative of the $V_T$ level set on the ventilator **20;** a breathing gas flow rate (V) signal indicative of the V level set on the ventilator **20;** a pressure limit signal indicative of the pressure limit set on the ventilator **20;** a work of breathing (WOB) signal indicative of the WOB level set on the ventilator **20;** a pressure support ventilation (PSV) signal indicative of the PSV level set on the ventilator **20;** a positive end expiratory pressure (PEEP) signal indicative of the PEEP level set on the ventilator **20;** a continuous positive airway pressure (CPAP) signal indicative of the CPAP level set on the ventilator **20;** and a fractional inhaled oxygen concentration (FIO2) signal indicative of the FIO2 level set on the ventilator **20.**

For example, the step of determining the desired level settings of the ventilator setting controls **30** of the ventilator **20** may comprise the steps of generating ventilation data **72** from the received output signals **51** in the processing subsystem **40** and applying at least a portion of the generated ventilation data **72** and the ventilator setting parameter signal **42** to the neural network **82** of the processing subsystem **40.** If desired, at least a portion of the output signals **51** may also be applied to the neural network **82** as ventilation data **72.**

In an alternative example, the step of determining the desired level settings of the controls 30 of the ventilator **20** may comprise the steps of generating ventilation data **72** from the received output signals **51** in the processing subsystem **40,** applying a set of decision rules in the rule based front-end **96** to at least a portion of the ventilation data **72** and the ventilator setting parameter signal **42** to classify the applied portions of the ventilation data **72** and the ventilator setting parameter signal **42,** selecting an appropriate neural network **82** to use, and applying a portion of the ventilation data **72** and the ventilator setting parameter signal **42** to the selected neural network **82** which will be used to determine the desired level settings of the ventilator setting controls **30.**

The ventilator monitor system **10** of the present invention may be implemented in one of many different configurations. For example, the ventilator monitor system 10 may be incorporated within a ventilator **20.** In an alternative example, the ventilator monitor system 10 may be a stand alone monitor that is operatively connected to the ventilator **20.**

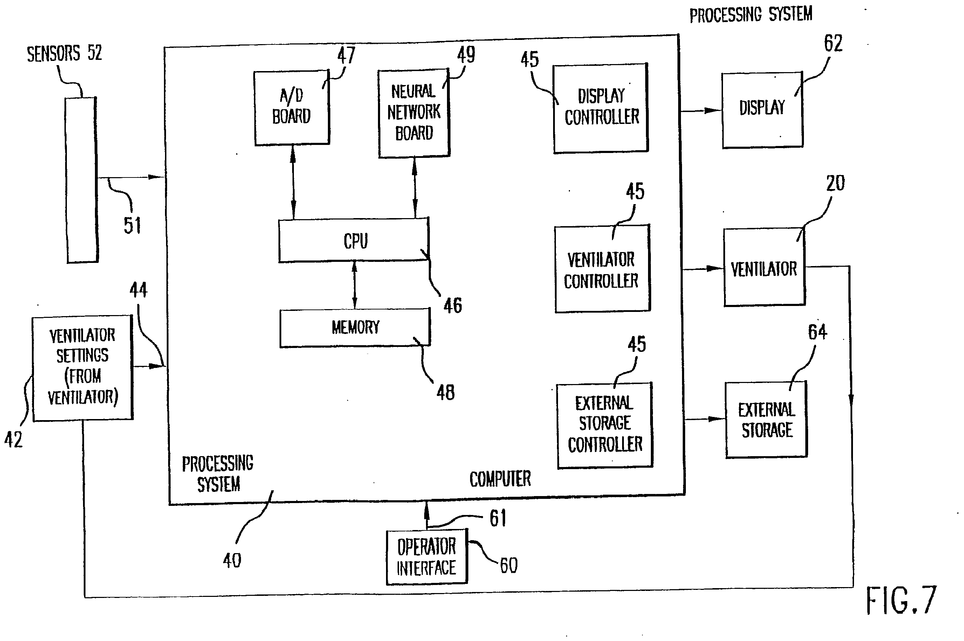

A realization of an embodiment of the processing subsystem **40** of the present invention is illustrated in Fig. 7. Here, the processing subsystem **40** includes the processor **46,** which is preferably a microprocessor, memory **48,** storage devices **64,** controllers **45** to drive the display **62,** storage **64,** and ventilator **20,** and an analog-to-digital converter (ADC) **47** if required. The processing subsystem **40** also includes a neural network **82,** which may, for example, be embodied in a neural network board **49**. The ADC and neural network boards **47, 49** are commercially available products. There is also an optional output board (not shown) for connection to a computer network and/or central monitoring station.

The ADC board **47** converts the analog signal received from the output of any of the sensors **52** of the measuring means to a digital output that can be manipulated by the processor **46**. In an alternative implementation, the output of any of the sensors **52** could be connected to the processor **46** via digital outputs, e.g., a serial RS232 port. The particular implementation is determined by the output features of the particular sensor **52**. The processor **46** should contain circuits to be programmed for performing mathematical functions, such as, for example, waveform averaging, amplification, linearization, signal rejection, differentiation, integration, addition, subtraction, division and multiplication, where desired. The processor **46** may also contain circuits to be programmed for performing neural/intelligent control software, neural network learning software, and ventilator control software, as required. Circuits or programs performing these functions are well known to one skilled in the art, and they form no part of the present invention. The processor **46** executes the software which makes the computations, controls the ADC and neural network boards **47, 49,** and controls output to the display and storage devices **62, 64,** network communication, and the ventilator apparatus **20**.

From a respiratory care standpoint, an example of the processing subsystems would include recommending new ventilator settings based on a mixture of rule-based respiratory therapy and derived parameters from the sensor inputs. For instance, to optimize patient effort, the system could use patient tidal volume and breathing frequency and also estimate patient effort using a mathematical model or neural network using wide variety of possible data from the sensors (potentially including the flow and pressure sensors, the pulse-oximeter/PPG, the CO2 sensor, and blood pressure signal). Additionally, a patient's tolerance for the breathing effort they are making may also be tracked using these same sensors. For example (but not limited to), tidal volume, peak inspiratory flow rate, breathing frequency, pulse rate and pulse rate changes.

In one embodiment, optimization of ventilation could be performed in accordance with the present invention by tracking changes in the CO2 sensor, patient deadspace and cardiac output (derived from the CO2 sensor and blood gases), and pulse-oximeter/PPG. Optimization of oxygenation occurs by tracking changes in oxygenation and the effect of PEEP on the cardiac system using a combination of a plethysmography and ExCO2 sensor output, potentially in combination with the following: airway pressure and flow sensors, and/or blood gases.

The purpose of the neural network board **49** is to implement the neural/intelligent control software. As one skilled in the art will appreciate, the need for a separate neural network board **49** is determined by the computational power of the main processor **46**. With recent increases in microprocessor speeds, it may not be necessary to have a separate board **49,** since some or all of these functions could be handled by the processor **46**. The need for the separate board **49** is also determined by the precise platform on which the invention is implemented.

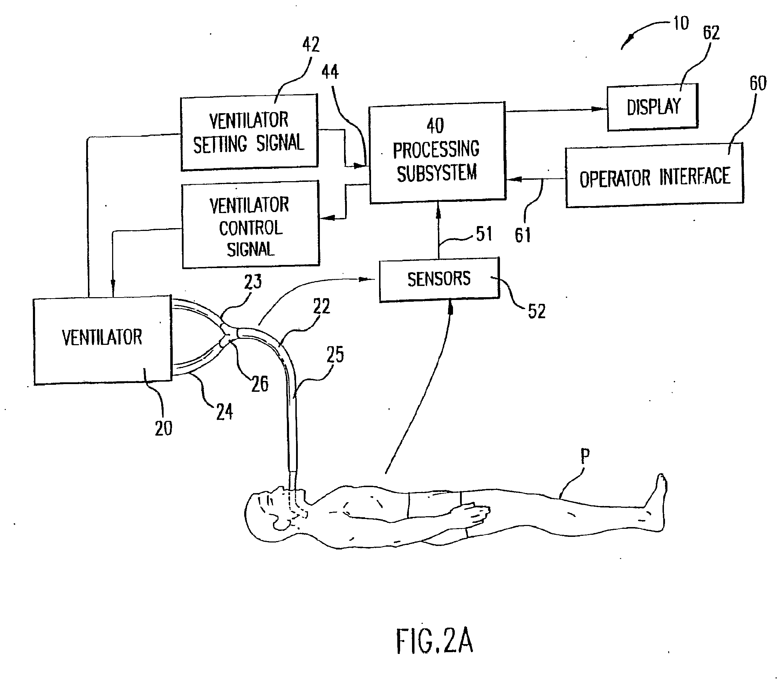

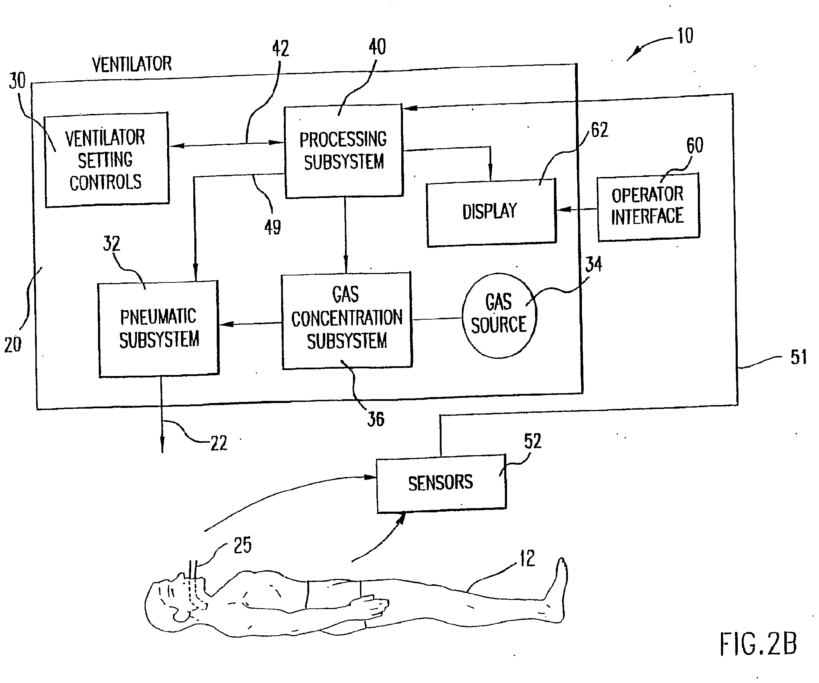

In addition, while the processor **46** of the processing subsystem **40** has been described as a single microprocessor, it should be understood that two or more microprocessors could be used dedicated to the individual functions. For example, the ventilator **20** may have a microprocessor that is operatively coupled to the processing subsystem **40** of the monitor system **10**. In this manner the monitor system **20** could be incorporated into a modular system **10** that may be coupled to any conventional microprocessor-controlled ventilator **20** for monitoring of the ventilation support provided by the ventilator **20**. Alternatively, as one skilled in the art will appreciate, and as shown in Fig. 2B, the monitor system **10** of the present invention may be incorporated into the design of a microprocessor-controlled ventilator **10** with the processing subsystem **40** of the ventilator monitor system using the microprocessor of the ventilator **20**. In addition, the functions of the processor **46** could be achieved by other circuits, such as application specific integrated circuits (ASIC), digital logic circuits, a microcontroller, or a digital signal processor.

The invention has been described herein in considerable detail, in order to provide those skilled in the art with information needed to apply the novel principles, and to construct and use such specialized components as are required. However, it is to be understood that the invention can be carried out by specifically different equipment and devices, and that various modification, both as to equipment details and operating procedures can be effected without departing from the scope of the invention itself. Further, it should be understood that, although the present invention has been described with reference to specific details of certain embodiments thereof, it is not intended that such details should be regarded as limitations upon the scope of the invention except as and to the extent that they are included in the accompanying claims. The present invention is limited solely by the scope of the appended claims.

**Claims**

1. A method for monitoring respiratory support for a patient having an airway, wherein said method comprises:

    (1) providing a monitoring system comprising:

(a) at least two sensors adapted to monitor the patient, or to monitor a patient airway access, each sensor generating an output signal, said output signals comprising at least a plethysmography output signal and an exhaled carbon dioxide (ExCO2) output signal;

(b) an operator interface that generates at least one operator input signal, and

(c) a processing subsystem adapted to receive the at least two of the output signals and at least one operator input signal, wherein the processing subsystem has a processor and a memory and is adapted to run under control of a program stored in the memory, wherein the processing subsystem evaluates at least two output signals and at least one operator input signal to determine a desired setting for at least one ventilation parameter and wherein the processing system comprises a neural network;

(2) receiving into the processing subsystem at least two of the output signals;

(3) implementing the processing subsystem to evaluate the at least two output signals and at least one operator input signal to assess respiratory support provided to the patient; and

(4) providing a recommendation by the monitoring system for the desired setting for at least one ventilation parameter based on the evaluation of the at least two output signals and at least one operator input signal by the processing subsystem, **characterized in that** the plethysmography output signal and the exhaled carbon dioxide (ExCO2) output signal are used by the neural network to track changes in oxygenation and the effect of PEEP on the cardiac system and thereby to recommend a desired setting for a positive end expiratory pressure (PEEP) signal to optimize oxygenation without sacrificing cardiac output.

2. The method of claim 1, further comprising deriving patient effort of breathing from the evaluation of the output signals and operator input signals; wherein the processing subsystem evaluates the patient effort of breathing and at least one parameter for use in recommending the desired setting.

3. The method of any of the preceding claims, wherein said output signals comprise at least one of the group consisting of: a flow rate signal indicative of the flow rate (V) of the inhaled/exhaled gas expired by the patient within the breathing circuit; a pressure (P) signal indicative of the pressure of the breathing gas within the breathing circuit; a blood pressure (BP) signal indicative of the blood pressure of the patient wherein the blood pressure (BP) signal is preferably derived from at least one of the group consisting of: exhaled carbon dioxide (ExCO2) signal; SpO2 signal; arterial line, PPG signal; pulse transit time/pulse wave velocity; and pulse pressure; and a temperature (T) signal indicative of the core body temperature of the patient.

4. The method of any of the preceding claims, wherein said output signals or at least one ventilation parameter also includes at least one of the group consisting of: an arterial blood gas PaO2 level of the patient; an arterial blood gas PaCO2 level of the patient; and an arterial blood gas pH level of the patient.

5. The method of any of the preceding claims, wherein the operator input signals comprise at least one of the group consisting of: patient identification information; patient diagnostic information; patient age; type and size of patient airway access; patient height; and patient weight.

6. The method of any of the preceding claims, wherein the processing subsystem (40) further comprises an intelligence subsystem, such as at least one neural network, preferably including means for training the neural network.

7. The method of any of the preceding claims, wherein the processing subsystem (40) further comprises a feature extraction subsystem.

8. The method of any of the preceding claims, wherein said at least one desired setting optimizes one of the following selected from the group consisting of: patient ventilation, oxygenation, and breathing effort.

9. The method of any of the preceding claims, wherein the processing subsystem (40) is able to evaluate time history of output signals and/or operator input signals for determining the desired setting.

**Patentansprüche**

1. Verfahren zum Überwachen einer Atemunterstützung eines Patienten mit einem Atemweg, wobei das Verfahren Folgendes umfasst:

(1) Bereitstellen eines Überwachungssystems, Folgendes umfassend:

(a) wenigstens zwei Sensoren, die geeignet sind, um den Patienten zu überwachen oder um einen Atemwegszugang des Patienten zu überwachen, wobei jeder Sensor ein Ausgangssignal erzeugt, wobei die Ausgangssignale wenigstens ein Plethysmographieausgangssignal und ein ausgeatmetes Kohlendioxid(*exhaled carbon dioxide* - ExCO2)ausgangssignal umfassen;
(b) eine Bedienschnittstelle, die wenigstens ein Bedieneingangssignal erzeugt, und
(c) ein Verarbeitungsteilsystem, das geeignet ist, um die wenigstens zwei der Ausgangssignale und wenigstens ein Bedieneingangssignal zu empfangen, wobei das Verarbeitungsteilsystem einen Prozessor und einen Speicher aufweist und geeignet ist, um unter Steuerung eines in dem Speicher gespeicherten Programms zu laufen, wobei das Verarbeitungsteilsystem wenigstens zwei Ausgangssignale und wenigstens ein Bedieneingangssignal auswertet, um eine gewünschte Einstellung für wenigstens einen Beatmungsparameter zu bestimmen, wobei das Verarbeitungssystem ein neuronales Netzwerk umfasst;

(2) Empfangen von wenigstens zwei der Ausgangssignale in das Verarbeitungsteilsystem;
(3) Implementieren des Verarbeitungsteilsystems, um die wenigstens zwei Ausgangssignale und wenigstens ein Bedieneingangssignal auszuwerten, um die dem Patienten bereitgestellte Atemunterstützung einzuschätzen; und
(4) Bereitstellen einer Empfehlung durch das Überwachungssystems für die gewünschte Einstellung für wenigstens einen Beatmungsparameter basierend auf der Auswertung der wenigstens zwei Ausgangssignale und wenigstens einem Bedieneingangssignal durch das Verarbeitungsteilsystems, **dadurch gekennzeichnet, dass** das Plethysmographieausgangssignal und das ausgeatmete Kohlendioxid(ExCO2)ausgangssignal von dem neuronalen Netzwerk verwendet werden, um Änderungen der Sauerstoffversorgung und die Wirkung von PEEP auf das Herzsystem zu verfolgen und dadurch eine gewünschte Einstellung für ein positives endexspiratorisches *Druck(positive end expiratory pressure* - PEEP)signal zu empfehlen, um die Sauerstoffversorgung zu optimieren, ohne dass Herzleistung geopfert wird.

2.  Verfahren nach Anspruch 1, ferner umfassend ein Herleiten der Atemanstrengung des Patienten aus der Auswertung der Ausgangssignale und der Bedieneingangssignale;
wobei das Verarbeitungsteilsystem die Atemanstrengung des Patienten und wenigstens einen Parameter zur Verwendung bei der Empfehlung der gewünschten Einstellung auswertet.

3.  Verfahren nach einem der vorhergehenden Ansprüche, wobei die Ausgangssignale wenigstens eines aus der Gruppe umfassen, die aus Folgendem besteht:

einem Durchflussmengensignal, das die Durchflussmenge (V) des eingeatmeten/ausgeatmeten Gases, das durch den Patienten innerhalb der Atemleitung exspiriert wird, anzeigt;
einem *Druck(pressure - P)*signal, das den Druck des Atemgases innerhalb der Atemleitung anzeigt;
einem Blutdruck(*blood pressure* - BP)signal, das den Blutdruck des Patienten anzeigt, wobei das Blut(BP)drucksignal vorzugsweise aus wenigstens einem der Gruppe hergeleitet ist, die aus Folgendem besteht:

ausgeatmeten Kohlendioxid(ExCO2)signal;
SpO2-Signal;
arterieller Linie, PPG-Signal;
Pulslaufzeit/Pulswellengeschwindigkeit; und
Pulsdruck; und
einem Temperatur(T)signal, das die Kernkörpertemperatur des Patienten anzeigt.

4.  Verfahren nach einem der vorhergehenden Ansprüche, wobei die Ausgangssignale oder wenigstens ein Beatmungsparameter ebenso eines der Gruppe einschließen, die aus Folgendem besteht:

einem arteriellen Blutgas-PaO2-Spiegel des Patienten;
einem arteriellen Blutgas-PaCO2-Spiegel des Patienten; und
einem arteriellen Blutgas-pH-Wertspiegel des Patienten.

5.  Verfahren nach einem der vorhergehenden Ansprüche, wobei die Bedieneingangssignale wenigstens eines aus der Gruppe umfassen, die aus Folgendem besteht:

Patientenidentifikationsinformationen;
Patientendiagnoseinformationen;
Patientenalter;
Art und Größe des Atemwegszugangs des Patienten;
Patientengröße; und
Patientengewicht.

6.  Verfahren nach einem der vorhergehenden Ansprüche, wobei das Verarbeitungsteilsystem (40) ferner ein Intelligenzteilsystem, wie etwa wenigstens ein neuronales Netz, vorzugsweise einschließlich Mittel zum Trainieren des neuronalen Netzwerks, umfasst.

**7.** Verfahren nach einem der vorhergehenden Ansprüche, wobei das Verarbeitungsteilsystem (40) ferner ein Merkmalextraktionsteilsystem umfasst.

**8.** Verfahren nach einem der vorhergehenden Ansprüche, wobei die wenigstens eine gewünschte Einstellung eines der Folgenden optimiert, die aus der Gruppe ausgewählt sind, die aus Folgendem besteht:
Beatmung, Sauerstoffversorgung und Atemanstrengung des Patienten.

**9.** Verfahren nach einem der vorhergehenden Ansprüche, wobei das Verarbeitungsteilsystem (40) in der Lage ist, einen Zeitverlauf der Ausgangssignale und/oder Bedieneingangssignale zum Bestimmen der gewünschten Einstellung auszuwerten.

**Revendications**

**1.** Procédé de surveillance de l'assistance respiratoire pour un patient muni d'une canule trachéale, dans lequel ledit procédé comprend :

(1) la fourniture d'un système de surveillance comprenant :

(a) au moins deux capteurs conçus pour surveiller le patient ou pour surveiller un accès des voies respiratoires du patient, chaque capteur générant un signal de sortie, lesdits signaux de sortie comprenant au moins un signal de sortie de pléthysmographie et un signal de sortie de dioxyde de carbone expiré (ExCO2) ;
(b) une interface opérateur qui génère au moins un signal d'entrée opérateur, et
(c) un sous-système de traitement conçu pour recevoir au moins deux des signaux de sortie et au moins un signal d'entrée d'opérateur, dans lequel le sous-système de traitement possède un processeur et une mémoire et est conçu pour fonctionner sous le contrôle d'un programme stocké dans la mémoire, dans lequel le sous-système de traitement évalue au moins deux signaux de sortie et au moins un signal d'entrée d'opérateur afin de déterminer un réglage souhaité pour au moins un paramètre de ventilation et dans lequel le système de traitement comprend un réseau neuronal ;

(2) la réception dans le sous-système de traitement d'au moins deux des signaux de sortie ;
(3) la mise en œuvre du sous-système de traitement pour évaluer les au moins deux signaux de sortie et au moins un signal d'entrée d'opérateur afin de mesurer l'assistance respiratoire fournie au patient ; et
(4) la fourniture d'une recommandation par le système de surveillance pour le réglage souhaité pour au moins un paramètre de ventilation en fonction de l'évaluation des au moins deux signaux de sortie et d'au moins un signal d'entrée de l'opérateur par le sous-système de traitement, **caractérisé en ce que** le signal de sortie de pléthysmographie et le signal de sortie de dioxyde de carbone expiré (ExCO2) sont utilisés par le réseau neuronal afin de suivre des changements d'oxygénation et l'effet de la PEP sur le système cardiaque et ainsi recommander un réglage souhaité pour un signal de pression expiratoire positive (PEP) afin d'optimiser l'oxygénation sans sacrifier le débit cardiaque.

**2.** Procédé selon la revendication 1, comprenant en outre la dérivation de l'effort respiratoire du patient à partir de l'évaluation des signaux de sortie et des signaux d'entrée de l'opérateur ; dans lequel le sous-système de traitement évalue l'effort respiratoire du patient et au moins un paramètre à utiliser afin de recommander le réglage souhaité.

**3.** Procédé selon l'une quelconque des revendications précédentes, dans lequel lesdits signaux de sortie comprennent au moins l'un du groupe constitué : d'un signal de taux d'écoulement indiquant le taux d'écoulement (V) du gaz inhalé/exhalé expiré par le patient à l'intérieur du circuit respiratoire ; d'un signal de pression (P) indiquant la pression du gaz respiratoire à l'intérieur du circuit respiratoire ; d'un signal de pression artérielle (BP) indiquant la pression artérielle du patient, le signal de pression artérielle (BP) étant de préférence dérivé d'au moins l'un du groupe constitué : d'un signal de dioxyde de carbone expiré (ExCO2) ; d'un signal SpO2 ; d'une ligne artérielle, d'un signal PPG ; d'un temps de transit d'impulsion/d'une vitesse d'onde d'impulsion ; et d'une pression d'impulsion ; et d'un signal de température (T) indiquant la température corporelle centrale du patient.

**4.** Procédé selon l'une quelconque des revendications précédentes, dans lequel lesdits signaux de sortie ou au moins un paramètre de ventilation comportent également au moins l'un du groupe constitué : d'un niveau de PaO2 de gaz du sang artériel du patient ; d'un niveau de PaCO2 de gaz du sang artériel du patient ; et d'un niveau de pH de gaz du sang artériel du patient.

**5.** Procédé selon l'une quelconque des revendications précédentes, dans lequel les signaux d'entrée de l'opérateur comprennent au moins l'un du groupe constitué : d'informations d'identification du patient ;

d'informations de diagnostique du patient ; de l'âge du patient ; du type et de la taille de l'accès des voies respiratoires du patient ; de la taille du patient ; et du poids du patient.

6. Procédé selon l'une quelconque des revendications précédentes, dans lequel le sous-système de traitement (40) comprend en outre un sous-système d'intelligence, tel qu'au moins un réseau neuronal, comportant de préférence un moyen destiné à former le réseau neuronal.

7. Procédé selon l'une quelconque des revendications précédentes, dans lequel le sous-système de traitement (40) comprend en outre un sous-système d'extraction de caractéristiques.

8. Procédé selon l'une quelconque des revendications précédentes, dans lequel ledit au moins un réglage souhaité optimise l'un des suivants sélectionné dans le groupe constitué de : la ventilation du patient, l'oxygénation et l'effort respiratoire.

9. Procédé selon l'une quelconque des revendications précédentes, dans lequel le sous-système de traitement (40) est capable d'évaluer l'historique temporel des signaux de sortie et/ou des signaux d'entrée de l'opérateur afin de déterminer le réglage souhaité.

FIG.1

FIG.2A

EP 2 029 209 B1

FIG.2B

FIG.3

EP 2 029 209 B1

FIG.4

EP 2 029 209 B1

FIG.5

24

80

72

NEURAL NETWORK

OUTPUT

82

75

42

FIG.6A

VENTILATOR
SETTING IMPUT

INTELIGENCE
SUBSYSTEM

OPERATOR INPUT

98

90

94

RULE
BASED
BACK
END

92

96

75 72

VENTILATION
STATUS
MODULE

RULE
BASED
FRONT
END

VENT SETTING
PREDICTOR

82

OUTPUT

MODULE
#1

82

82

MODULE
#2

82

42

MODULE
#N

82

80

INTELLIGENCE
SUBSYSTEM

USER INPUT

FIG.6B

VENTILATOR
SETTING INPUT

42

SENSORS 52

A/D BOARD 47

NEURAL NETWORK BOARD 49

DISPLAY CONTROLLER 45

DISPLAY 62

51

CPU

46

VENTILATOR CONTROLLER 45

VENTILATOR 20

VENTILATOR SETTINGS (FROM VENTILATOR) 42

44

MEMORY

48

EXTERNAL STORAGE CONTROLLER 45

EXTERNAL STORAGE 64

PROCESSING SYSTEM

COMPUTER

40

OPERATOR INTERFACE 60

61

FIG.7

FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0100265 A
- WO 2004019766 A
- US 6431171 B
- US 4838259 A, Gluck
- US 5303698 A, Tobia
- US 5400777 A, Olsson
- US 5429123 A, Shaffer
- US 5692497 A, Schnitzer
- US 6648831 B

**Non-patent literature cited in the description**

- **RUMELHARDT et al.** Parallel Distributed Processing: Explorations in the Microstructure of Cognition. MIT Press, 1986, vol. 1-2